# EUROPÄISCHE PATENTSCHRIFT

(11) **EP 3 393 414 B1**
(45) Veröffentlichungstag und Bekanntmachung des Hinweises auf die Patenterteilung: **02.06.2021**
(21) Anmeldenummer: 16820290.1
(22) Anmeldetag: 23.12.2016
(51) Int. Cl.: A61F 13/00, A61F 13/02, A61F 13/06, A61F 13/08, D04B 21/16

(54) **KOMPRESSIONSVERBAND**
COMPRESSION DRESSING
BANDAGE DE COMPRESSION

(30) Priorität: 23.12.2015 DE 102015226706
(43) Veröffentlichungstag der Anmeldung: 31.10.2018
(73) Patentinhaber: KOB GmbH, 67752 Wolfstein (DE)
(72) Erfinder: LUDWIG, Uwe, 67752 Wolfstein (DE); TAMOUE, Ferdinand, 67071 Ludwigshafen am Rhein (DE); MEISTER, Marita, 67657 Kaiserslautern (DE)
(74) Vertreter: DREISS Patentanwälte PartG mbB
(86) Internationale Anmeldenummer: PCT/EP2016/082626
(87) Internationale Veröffentlichungsnummer: WO 2017/109209

(56) Entgegenhaltungen:
- EP-A1- 2 698 135
- EP-A2- 2 275 062
- WO-A1-2013/001312
- DE-A1- 19 746 913
- US-A- 4 998 421

## Beschreibung

Die Erfindung betrifft einen Kompressionsverband umfassend zwei Schichten.

Kompressionsverbände werden im Stand der Technik beispielsweise für diabetische Ulzera eingesetzt. Hierbei besteht das Problem, dass aufgrund des Krankheitsbildes notwendigerweise verschiedene Fragestellungen zu berücksichtigen sind, nämlich zum einen eine hinreichende Kompression, zum anderen aber auch eine Polsterwirkung gegenüber der zu behandelnden Gliedmaße.

So kann es beispielsweise vorgesehen sein, eine Polsterung aufzubringen und darüber eine Kompressionsbinde anzulegen.

Dabei sind bei der Kompressionstherapie grundsätzlich der sogenannte Arbeitsdruck und der sogenannte Ruhedruck zu unterscheiden, wobei der Ruhedruck der Druck ist, der bei liegender Gliedmaße durch das Kompressionsmittel, hier den Kompressionsverband, auf die Gliedmaße ausgeübt wird. Der Arbeitsdruck ist dann der Druck, der auf die Gliedmaße bei Bewegung der Muskeln ausgeübt wird. Bevorzugt soll der Arbeitsdruck 20 bis 40 mm Hg über dem Ruhedruck liegen.

Es ist dabei ebenfalls bekannt, verschiedene Bindentypen einzusetzen. So sind sogenannte Langzugbinden bekannt, die eine sehr hohe Elastizität aufweisen und häufig eine Dehnfähigkeit über 200 % besitzen, wobei demgegenüber Kurzzugbinden bekannt sind, die nur eine geringe Dehnbarkeit besitzen und nur eine geringe Rückstellkraft, jedoch bereits sehr früh keine weitere Dehnung zulassen und so einen vergleichsweise hohen Arbeitsdruck aufzubauen vermögen. Demgegenüber bilden Kurzzugbinden über einen vergleichsweise langen Bereich nur einen geringen Widerstand auf, um dann eine sehr hohe Begrenzung der Dehnbarkeit zu realisieren. Die traditionelle Kurzzugbandagenkompressionstherapie ist eine Maßnahme für die Behandlung von Venenerkrankungen. Dabei werden die Materialien für Kurzzugbinden in der Regel aus nicht elastischen Materialien gefertigt und durch Ausrüstungsprozesse elastifiziert. Die Elastizität reduziert sich jedoch während der Behandlung signifikant. Dies kann zu einer Reduktion des Kompressionsdrucks in der Anwendung führen.

Bekannte Kompressionsbandagen sind beispielsweise in der EP 2 275 062 A2 beschrieben, die eine innere der Haut zugewandte elastische Bandage beschreibt mit einem gedehnten elastischen Substrat und einer gedehnten Schaumschicht, die auf der hautzugewandten Seite des Substrats angeordnet ist, sowie einer weiteren gedehnten selbstklebenden elastischen Bandage, die hierüber angelegt wird.

Darüber hinaus ist ein Stütz-, Fixier- oder Druckverband aus der DE 20 2012 000 529 U1 bekannt, wobei der Verband hier mindestens vier Lagen aufweist und eine Spannlage besitzt, die die Rückstellkraft erzeugt und Löcher aufweist, wobei die übrigen Lagen durch die Löcher miteinander fixiert sind. Durch die vier vorgesehenen Lagen ist der Verband vergleichsweise teuer und aufwendig.

Des Weiteren ist noch auf die WO 2014/131976 A2 hinzuweisen, die ebenfalls eine elastische Bandage betrifft, umfassend eine nicht elastische Schicht, die die elastische Bande umschließt und hiermit verbunden ist.

Zur Erreichen einer hohen therapeutischen Sicherheit ist es wünschenswert, dass der therapeutisch notwendige Kompressionsdruck möglichst gut erreicht wird. Hierzu sind im Stand der Technik eine Vielzahl von Möglichkeiten bekannt. So sind beispielsweise Markierungsmittel bekannt, die bei einer zu großen Dehnung eine Verformung erfahren, so dass der Therapeut eine zu große Dehnung und damit einen zu hohen Anlegedruck erkennen kann. Nachteilig ist jedoch dabei, dass hierfür stets geschultes Personal notwendig ist.

Des Weiteren ist ein Verfahren zum Verbinden zweier Bindenlagen zur Bildung eines Kompressionsverbands beispielsweise mittels Haftpunkten oder Schweißpunkten aus der EP 2 698 135 A1 vorbekannt. Die grundsätzliche Verwendung von Nähwirkverfahren ist z. B. aus den Dokumenten DE 197 46 913 A1 sowie US 4,998,421 für Binden bekannt.

Es ist daher wünschenswert, einen Kompressionsverband, der alternativ auch als Kompressionsbinde oder -bandage bezeichnet werden kann, bereitzustellen, der eine hohe Anlegesicherheit aufweist bei gleichzeitig guten therapeutischen Eigenschaften und der insbesondere über mehrere Tage getragen werden kann, ohne dass ein deutlicher Abfall des Kompressionsdrucks festzustellen ist.

Sofern die Begriffe Schicht oder Lage verwendet werden, bezeichnen diese den gleichen Gegenstand.

Ausgehend von diesem Stand der Technik löst die Erfindung die Aufgabe durch einen Kompressionsverband mit den Merkmalen des Anspruchs 1, wobei die beiden Schichten, nämlich die erste Polsterschicht und die zweite Stützschicht, mittels Nähwirkverfahren über einen elastischen Faden miteinander verbunden sind und die Stichlänge 1,5 bis 3 mm/u beträgt bei einer Nähfadenspannung von höchstens 4 cN.

Durch diese Gestaltung kann erreicht werden, dass die beiden Schichten mittels des Nähwirkverfahrens und hier vorzugsweise einem Malimo- bzw. Maliwatt-Verfahren verbunden werden, wobei die Schichten im ungedehnten Zustand mittels des elastischen Nähfadens verbunden werden.

Der Vorteil bei einem Nähwirkverfahren besteht darin, dass gleichzeitig an mehreren Stellen eine Verbindung durchgeführt werden kann und die beiden Schichten nach der Verbindung nicht mehr voneinander getrennt werden können. Über die Auswahl der Stichlänge in Längsrichtung des Flächengebildes aus dem dann die Kompressionsbinden konfektioniert werden, wobei unter Stichlänge der Abstand in Stichlängsrichtung zwischen zwei Stichen verstanden werden soll, und der Nähfadenspannung die Dehnbarkeit des elastischen Verbundes eingestellt werden kann, so dass ein von Hand nicht mehr trennbarer und trotzdem kontrollierbar elastischer Verbund aus den beiden Schichten besteht. Je nach Auswahl dieser Parameter zieht sich das fertige Flächengebilde zusammen bei Entspannung und werden Falten im Material aufgeworfen.

Die Nähtechnik und die Stichlänge des Nähfadens werden dabei so reguliert, dass die Fasern auf der Polsterschicht auf der einen Seite des Verbundes aus den zwei Schichten Hautkomfort und Ausgleichsfunktionen besitzen und damit letztendlich der Kompressionsverband zwei erkennbar unterschiedliche Seiten aufweist, die für den Druckausgleich sehr funktional sind. Des Weiteren ist die Stichlänge dabei so einzustellen, dass die gewünschten absorbierenden sowie hautfreundlichen Eigenschaften der der Haut zugewandten Polsterschicht erhalten bleiben.

Die Polsterschicht ist dabei die einer Gliedmaße zugewandte Seite eines Kompressionsverbandes und die Stützschicht die hierauf aufgebrachte zweite Seite.

Das Malimo- bzw. Maliwattverfahren zum Verbinden der Schichten wird dabei, wie im Stand der Technik bekannt eingesetzt. So kann z.B. die Elastizität durch Übernähen eines starren Vliessstoffes oder Gewebes mit dauerelastischen Elastanfäden in Längsrichtung unter Anwendung der Nähwirktechnik MALIWATT oder Malimo erhalten wurde. Die Nähwirktechnik MALIWATT bzw. Malimo ist in Malimo Nähwirktechnologie, Ploch, Böttcher, Scharch, VEB Fachbuchverlag Leipzig, 1978, 1. Auflage beschrieben.

Bevorzugt können beide Schichten, also die Polsterschicht und die Stützschicht unelastisch ausgebildet sind und werden erst durch das Nähwirkverfahren elastifiziert. Besonders bevorzugt kann vorgesehen sein, dass mindestens eine der beiden Schichten, jedoch bevorzugt beide der Schichten aus Polstermaterial und Stützschicht ein Vliesmaterial sind. Alternativ sind jedoch auch andere Materialien wie z.B. Gewebe, Gewirke, Gestricke oder Schäume einsetzbar. Dabei kann es sich bei der Polsterschicht um eine Wattevliesschicht, insbesondere eine Thermofusionsvliesschicht, handeln, die gegebenenfalls auch schon vorvernadelt sein kann. Dabei werden bei beiden Verfahren, nämlich dem Thermobonding als auch der der Thermofusion die Fasern der Vliese in einem Kämmereiverfahren in eine bestimmte Richtung gelegt und in einem Textilfunktionalisierungsverfahren als Vliesrollen vorbereitet und durch Temperatur oder durch Temperatur und Druck stabilisiert für die Weiterverarbeitung. Während des Thermofusionsverfahrens werden Fasern mit verschiedenen Schmelzpunkten durch Heißluft-Trockner miteinander verschmolzen. Im Thermobondingverfahren werden die Fasern mittels Hitze und Druck zwischen beheizten Kalanderrollen verschmolzen. Das Ergebnis sind in beiden Fällen weiche, homogene Vliesstoffe, die ideal und für technische Anwendungen geeignet sind. Das Thermofusionsverfahren eignet sich aufgrund des fehlenden Drucks besser für Polsterschichten.

Bei der Stützschicht kann es sich um ein Thermobondvlies handeln. Das Thermobondvlies weist dabei vorzugsweise lediglich eine geringe Dehnfähigkeit bei gleichzeitig gewünschter Steifigkeit auf.

Die beiden Schichten werden zusammen einer Kettenwirkmaschine zugeführt und mittels eines elastischen Nähfadens, der vorzugsweise aus einer Gruppe aus Baumwollspinnkreppfäden, Baumwollzwirnkreppfäden, texturierten Polyamidgarnen, texturierten Polyestergarnen, Gummifäden oder Polyurethanelastanfäden oder einer Kombination hiervon ausgewählt werden kann, miteinander verbunden.

Der Nähfaden kann alternativ auch als Kettfaden bezeichnet sein. Der Faden verläuft dabei in Maschinenrichtung der Kettenwirkmaschine und nicht quer hierzu.

Das fertig vernähte Flächengebilde des Kompressionsverbandes weist stets eine optimierte Dehnung auf, wobei besonders bevorzugt vorgesehen sein kann, dass die maximale Dehnbarkeit des Kompressionsverbandes, der einer vorgegebenen optimalen Dehnbarkeit entspricht, und eine hierüber hinausgehende Dehnung des Kompressionsverbandes durch eine Dehnungsschwelle begrenzt ist. Auf diese Weise kann die Anlegersicherheit signifikant erhöht werden, da es auch für nicht geübte Anwender möglich ist, den Kompressionsverband maximal bis zur Dehnungsschwelle zu dehnen, wobei dann nicht nur die maximale Dehnbarkeit, sondern zugleich auch die optimale Dehnung und damit der optimale Kompressionsdruck erreicht ist und in diesem maximal gedehnten Zustand den Kompressionsverband anzulegen.

Um eine optimale Polsterwirkung zu erzielen, kann es bevorzugt vorgesehen sein, dass die Dicke der Polsterschicht 0,3-12 mm, bevorzugt 0,4-6 mm und weiter bevorzugt 0,5-3 mm, besonders bevorzugt 0,6-1,2mm beträgt.

Aufgrund der Verbindung mittels eines Nähwirkverfahrens werden die beiden Schichten im entspannten Zustand, nachdem das vernähte Flächengebilde mittels Längskonfektionierung zum Kompressionsverband weiterverarbeitet wurde, so in Wellen gelegt, dass eine unregelmäßige Oberfläche des Kompressionsverbandes entsteht. Aufgrund dieser unregelmäßigen Oberfläche wird neben der primären Funktion als Ausgleichslage des Kompressionsverbands und der sekundären Funktion der regulierbaren Dehnbarkeit und damit erhöhten Anlegesicherheit auch erreicht, dass durch die Wellen ein Muster auf der Oberfläche entsteht aus Materialerhebungen und Materialvertiefungen, dass auch im maximal gedehnten Zustand nicht vollständig aufgehoben ist, so dass in der Therapie hierdurch zusätzlich ein Massage- bzw. Drainageeffekt entsteht.

Zur Aufbringung eines Ruhedrucks kann darüber hinaus vorgesehen sein, die erfindungsgemäße Kompressionsbandage (Kompressionsverband) mit einer weiteren Kompressionsbandage zu kombinieren, insbesondere zu überwickeln, die z.B. als Langzugbandage ausgebildet sein kann, das heisst, von Beginn der Dehnung an einen Kompressionsdruck bereitzustellen, der jedoch nicht sprunghaft ansteigt.

Als weitere Bandage für den Ruhedruck wird bevorzugt eine elastische, kohäsiv haftende Vliesbinde vom Typ 752, Handelsname NOWOPRESS 752, Hersteller: Karl Otto Braun GmbH & Co. KG, Wolfstein, Deutschland als Kompressionsbinde mit Langzugeigenschaften verwendet, wobei das Grundtextil durch Übernähen eines starren Polypropylenvliesstoffs mit dauerelastischen Elastanfäden in Längsrichtung unter Anwendung der Nähwirktechnik MALIWATT erhalten wurde. Die Nähwirktechnik MALIWATT ist in Malimo Nähwirktechnologie, Ploch, Böttcher, Scharch, VEB Fachbuchverlag Leipzig, 1978, 1. Auflage beschrieben. Diese Bindenlage ist auf beiden Oberflächen mit einem kohäsiv haftenden Klebemittel auf Basis Polyisopren-Kautschuk beschichtet, um den gewünschten kohäsiven Hafteffekt zu erreichen. Die Bindenlage LS (Typ 752) ist dabei wie folgt aufgebaut:
60 % Polypropylen ,12 % Elastan, 28 % IRKautschuk Grund-Vlies: PP-Spunbondvlies , 35 g/qm , thermisch geprägt Nähfaden 133 dtex Elastan (DORLASTAN, BAYER) Nähfadendichte 45 Fäden je 10 cm Breite Nähfaden-Stichlänge, Bindung 3 mm, offene Franse Flächengewicht gedehnt 59 g/qm Elastizität in Längsrichtung (Kettrichtung) Dehnbarkeit / Rückzug nach DIN 61632 160 % / 99 % Haftkraft Seite A/B 60 cN/cm.

Die Erfindung betrifft daher auch einen Kombinationsverband aus der beschriebenen Bandage mit Polster- und Stützschicht und einer weiteren Bandage, die insbesondere als Langzugbinde für den Ruhedruck ausgebildet ist.

Die Einteilung in die Kategorien Kurz-, Mittel- oder Langzugbinde erfolgt je nach Dehnbarkeit und kann z.B. P. Asmussen, B. Söllner, Kompressionstherapie Prinzipien und Praxis, Verlag Urban & Fischer in Elsevier, 2004 auf Seite 121 entnommen werden. Die Dehnbarkeiten werden dabei nach DIN 61632 bestimmt.

Die Erfindung wird im Folgenden anhand einer Zeichnung näher beschrieben. Weitere Vorteile und Merkmale der Erfindung ergeben sich darüber hinaus aus den übrigen Anmeldungsunterlagen.

In der Zeichnung zeigt:
- Figur 1: ein Herstellverfahren eines derartigen Kompressionsverbandes und
- Figur 2: ein Kraftdehnungsdiagramm für einen fertigen Kompressionsverband der erfindungsgemäßen Art.

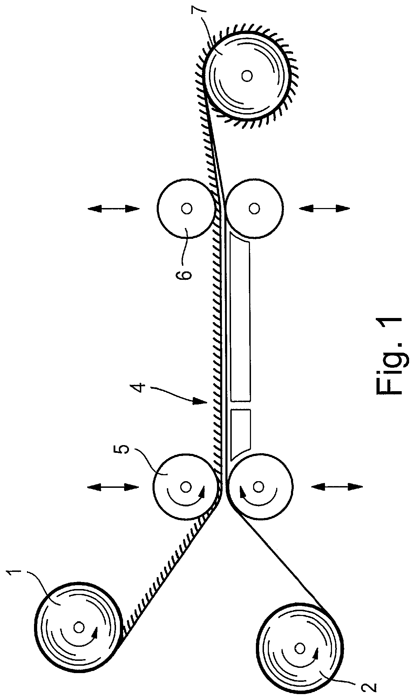

In Figur 1 ist dargestellt, wie zum einen eine Polsterschicht 1 aus einem Wattevlies und einer Stützschicht 2 hier als Thermobondvliesschicht aus einem Rollenmaterial zugeführt werden und in einem Nähwirkverfahren mittels eine Kettenwirkmaschine, die mit dem Bezugszeichen 4 versehen ist, miteinander verbunden werden. Dabei werden zuvor über eine Rollenführung 5 die beiden Lagen aufeinander fixiert. Das Nähwirkverfahren arbeitet dabei mit einem Haken, mittels dem die Schichten mit dem elastischen Nähfäden übernäht werden.. Das miteinander über den elastischen Nähfaden verbundene Material wird dann durch eine weitere Rollenführung 6 geführt und auf eine Rolle 7 aufgewickelt, wobei gegebenenfalls vorab eine Konfektionierung in Längsrichtung zu Binden erfolgen kann. Bei dem Nähfaden handelt es sich um einen elastisch vorgedehnten Faden, der mit einer vorgegebenen Stichlänge und einer vorgegebenen Nähfadenspannung eingebracht wird, und so nach Entspannung des elastischen Materials zu einem Zusammenziehen des Lagenverbundes (Flächengebildes) führt.

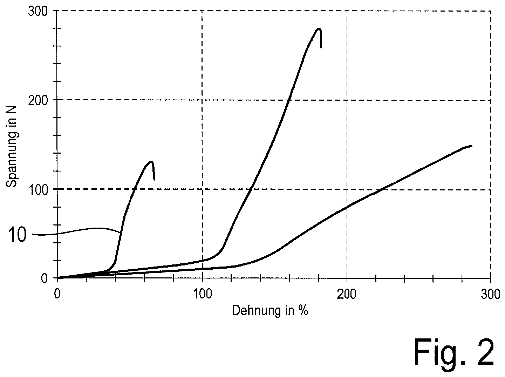

Figur 2 zeigt das Kraftdehnungsdiagramm, wobei der Kompressionsverband der Charakteristik einer Kurzzugbinde entspricht, die zunächst annähernd keine Krafterhöhung bei Dehnung aufweist, um dann einen sprunghaften Anstieg und damit eine Dehnungsschwelle zu realisieren, wobei die Dehnungsschwelle hier im Bereich des optimalen Therapiedrucks liegt, so dass bei maximaler Dehnung der Kompressionsbinde gleichzeitig bei Anlegen unter dieser Vorspannung der optimale Therapiedruck für die Kompressionstherapie eingestellt werden kann.

Die Steifigkeit einer Binde kann durch den Elastizitätsmodul und durch die Kraftsteigerung beschrieben werden. Figur 2 zeigt Kraft-Dehnungs-Diagramme der erfindungsgemäßen Binde 10 (im Vergleich mit zwei kommerziell erhältlichen Binden. Dabei weist die erfindungsgemäße Binde einen deutlich höheren Elastizitätsmodul auf als die Vergleichsbinden. Die bevorzugten Werte können 4-10 N/mm², bevorzugt 5-9 N/mm² und besonders bevorzugt 6-8 N/mm² betragen.

Bei der Kraftsteigerung handelt es sich darum, welche Dehnung die Binde vollziehen muss, um von einem signifikant spürbaren Widerstand von 20N bis zum Zerreißen gedehnt zu werden. Auch dieses Verhalten ist aus dem Diagramm gemäß Figur 2 zu erkennen. Bei der erfindungsgemäßen Binde wird eine Kraft von ca. 20N bei einer Dehnung von etwa 40% erreicht, die Binde reißt bei einer Dehnung von etwa 60%. In dem Bereich zwischen 40 und 60% Dehnung erfährt die Binde einen Kraftzuwachs um das fünf- bis zehn Fache. Bei den Vergleichsprodukten wird eine deutlich stärkere Dehnung bis zum Zerreißen notwendig. Bevorzugt weißt daher eine erfindungsgemäße Binde (Kompressionsverband) eine Dehnungszunahme von weniger als 50%, bevorzugt weniger als 35%, besonders bevorzugt weniger als 25% auf. Die Begriffe Kraftsteigerung und Dehnungszunahme beschreiben synonym den gleichen Sachverhalt.

## Patentansprüche

1. Kompressionsverband umfassend eine erste Polsterschicht (1) und eine zweite Stützschicht (2), **dadurch gekennzeichnet, dass** die beiden Schichten (1,2) im ungedehnten Zustand mittels Nähwirkverfahren über einen elastischen Nähfaden miteinander verbunden sind und die Stichläge 1,5 bis 3 mm/U beträgt bei einer Nähfadenspannung von höchstens 4 cN.

2. Kompressionsverband nach Anspruch 1, **dadurch gekennzeichnet, dass** die Dicke der Polsterschicht (1) 0,6-1,2 mm beträgt,

3. Kompressionsverband nach Anspruch 1 oder 2, **dadurch gekennzeichnet, dass** das Material einer oder beider der Schichten (1, 2) unelastisch ist.

4. Kompressionsverband nach einem der vorangehenden Ansprüche, **dadurch gekennzeichnet, dass** mindestens eine der beiden Schichten (1,2) aus Polsterschicht (1) und Stützschicht (2) aus einem Vliesmaterial bestehen.

5. Kompressionsverband nach Anspruch 4, **dadurch gekennzeichnet, dass** die Polsterschicht (1) ein Thermofusionsvlies ist.

6. Kompressionsverband nach einem der vorangehenden Ansprüche, **dadurch gekennzeichnet, dass** die zweite Stützschicht (2) ein Thermobondvlies ist.

7. Kompressionsverband nach einem der vorangehenden Ansprüche, **dadurch gekennzeichnet, dass** die elastischen Nähfäden aus einer Gruppe von BaumwollSpinnkreppfäden, Baumwoll-Zwirnkreppfäden, texturierten Polyamidgarnen, texturierten Polyestergarnen, Gummifäden oder PolyurethanElastanfäden oder einer Kombination hieraus ausgewählt werden.

8. Kompressionsverband nach einem der vorangehenden Ansprüche, **dadurch gekennzeichnet, dass** der Kompressionsverband eine Dehnungsschwelle aufweist, die die maximale Dehnbarkeit anzeigt.

9. Kompressionsverband nach einem der vorangehenden Ansprüche, **dadurch gekennzeichnet, dass** die maximale Dehnbarkeit einem therapeutisch vorgegebenen Anlegedruck des Kompressionsverbandes an eine Gliedmaße eines Trägers entspricht.

10. Kompressionsverband nach einem der vorangehenden Ansprüche, **dadurch gekennzeichnet, dass** der Kompressionsverband ein Elastizitätsmodul von 4-10 N/mm², bevorzugt 5-9 N/mm² und besonders bevorzugt 6-8 N/mm² aufweist.

11. Kompressionsverband nach einem der vorangehenden Ansprüche, **dadurch gekennzeichnet, dass** der Kompressionsverband eine Dehnungszunahme von weniger als 50%, bevorzugt weniger als 35%, besonders bevorzugt weniger als 25% aufweist.

12. Kompressionsverbandzusammenstellung umfassend einen ersten Kompressionsverband nach einem der vorangehenden Ansprüche sowie einen zweiten Kompressionsverband, der insbesondere über den ersten Kompressionsverband anlegbar ist.

13. Kompressionsverbandzusammenstellung nach Anspruch 12, **dadurch gekennzeichnet, dass** der erste Kompressionsverband einen Arbeitsdruck und der zweite Kompressionsverband einen Ruhedruck im angelegten Zustand auf eine Gliedmasse ausübt.

## Claims

1. A compression bandage comprising a first cushioning layer (1) and a second supporting layer (2), **characterized in that** the two layers (1, 2) are connected to one another in the unstretched state via an elastic sewing thread by means of stitch-bonding methods and the stitch length is 1.5 to 3 mm/revolution at a sewing thread tension of not more than 4 cN.

2. The compression bandage as claimed in claim 1, **characterized in that** the thickness of the cushioning layer (1) is 0.6-1.2 mm.

3. The compression bandage as claimed in claim 1 or 2, **characterized in that** the material of one or both of the layers (1, 2) is nonelastic.

4. The compression bandage as claimed in any of the preceding claims, **characterized in that** at least one of the two layers (1, 2) composed of cushioning layer (1) and supporting layer (2) consists of a nonwoven material.

5. The compression bandage as claimed in claim 4, **characterized in that** the cushioning layer (1) is a thermofusion nonwoven.

6. The compression bandage as claimed in any of the preceding claims, **characterized in that** the second supporting layer (2) is a thermobond nonwoven.

7. The compression bandage as claimed in any of the preceding claims, **characterized in that** the elastic sewing threads are selected from a group composed of cotton spun crepe threads, cotton twisted crepe threads, textured polyamide yarns, textured polyester yarns, rubber threads or polyurethane elastane threads or a combination thereof.

8. The compression bandage as claimed in any of the preceding claims, **characterized in that** the compression bandage has a stretching threshold which indicates the maximum stretchability.

9. The compression bandage as claimed in any of the preceding claims, **characterized in that** the maximum stretchability corresponds to a therapeutically predefined application pressure of the compression bandage on a limb of a wearer.

10. The compression bandage as claimed in any of the preceding claims, **characterized in that** the compression bandage has an elastic modulus of 4-10 N/mm², preferably 5-9 N/mm² and particularly preferably 6-8 N/mm².

11. The compression bandage as claimed in any of the preceding claims, **characterized in that** the compression bandage has an increase in stretch of less than 50%, preferably less than 35%, particularly preferably less than 25%.

12. A compression bandage combination comprising a first compression bandage as claimed in any of the preceding claims and also a second compression bandage which is, in particular, applicable over the first compression bandage.

13. The compression bandage combination as claimed in claim 12, **characterized in that** the first compression bandage exerts a working pressure on a limb in the applied state and the second compression bandage a resting pressure.

## Revendications

1. Bandage de compression comprenant une première couche de rembourrage (1) et une deuxième couche de support (2), **caractérisé par le fait que** les deux couches (1, 2) sont reliées l'une à l'autre à l'état non étiré au moyen d'un procédé de couture-tricotage par l'intermédiaire d'un fil élastique à coudre et que la longueur de point est comprise entre 1,5 et 3 mm/tr avec une tension de fil à coudre de 4 cN tout au plus.

2. Bandage de compression selon la revendication 1, **caractérisé par le fait que** l'épaisseur de la couche de rembourrage (1) est comprise entre 0,6 et 1,2 mm,

3. Bandage de compression selon la revendication 1 ou 2, **caractérisé par le fait que** le matériau de l'une ou des deux couches (1, 2) est inélastique.

4. Bandage de compression selon l'une quelconque des revendications précédentes, **caractérisé par le fait que** l'une au moins des deux couches (1, 2) parmi la couche de rembourrage (1) et la couche de support (2) est constituée d'un matériau non tissé.

5. Bandage de compression selon la revendication 4, **caractérisé par le fait que** la couche de rembourrage (1) est un non tisée de thermofusion.

6. Bandage de compression selon l'une quelconque des revendications précédentes, **caractérisé par le fait que** la deuxième couche de support (2) est un non-tissé thermo-lié.

7. Bandage de compression selon l'une quelconque des revendications précédentes, **caractérisé par le fait que** les fils élastiques à coudre sont choisis dans un groupe de fils crêpe de filage en coton, de fils crêpe retors en coton, de fils polyamide texturés, de fils polyester texturés, de fils de caoutchouc ou de fils de polyuréthane-élasthanne ou d'une combinaison de ceux-ci.

8. Bandage de compression selon l'une quelconque des revendications précédentes, **caractérisé par le fait que** le bandage de compression présente un seuil d'expansion qui indique l'extensibilité maximale.

9. Bandage de compression selon l'une quelconque des revendications précédentes, **caractérisé par le fait que** l'extensibilité maximale correspond à une pression d'application thérapeutiquement prédéterminée du bandage de compression sur un membre d'un porteur.

10. Bandage de compression selon l'une quelconque des revendications précédentes, **caractérisé par le fait que** le bandage de compression présente un module d'élasticité de 4 à 10 N/mm², de préférence de 5 à 9 N/mm² et de manière particulièrement préférée de 6 à 8 N/mm².

11. Bandage de compression selon l'une quelconque des revendications précédentes, **caractérisé par le fait que** le bandage de compression présente une augmentation d'extension inférieure à 50 %, de préférence inférieure à 35 %, de manière particulièrement préférée inférieure à 25 %.

12. Ensemble de bandage de compression comprenant un premier bandage de compression selon l'une quelconque des revendications précédentes et un deuxième bandage de compression qui, en particulier, peut être appliqué sur le premier bandage de compression.

13. Ensemble de bandage de compression selon la revendication 12, **caractérisé par le fait que**, à l'état appliqué, le premier bandage de compression exerce une pression de travail et le deuxième bandage de compression exerce une pression de repos sur un membre.
